Europäisches Patentamt

European Patent Office

Office européen des brevets

(19)

(11) Veröffentlichungsnummer : **0 460 123 B1**

(12) # EUROPÄISCHE PATENTSCHRIFT

(45) Veröffentlichungstag der Patentschrift :
09.06.93 Patentblatt 93/23

(21) Anmeldenummer : 90915458.5

(22) Anmeldetag : 19.09.90

(86) Internationale Anmeldenummer :
PCT/EP90/01585

(87) Internationale Veröffentlichungsnummer :
WO 91/08730 27.06.91 Gazette 91/14

(51) Int. Cl.⁵ : $A61K\ 7/00$, A61K 7/06,
A61K 7/11, A61K 7/32

(54) **LÖSUNGSMITTELGEMISCH UND KOSMETISCHES MITTEL MIT EINEM GEHALT AN DIESEM LÖSUNGSMITTELGEMISCH.**

(30) Priorität : 16.12.89 DE 3941572

(43) Veröffentlichungstag der Anmeldung :
11.12.91 Patentblatt 91/50

(45) Bekanntmachung des Hinweises auf die Patenterteilung :
09.06.93 Patentblatt 93/23

(84) Benannte Vertragsstaaten :
DE ES FR GB IT

(56) Entgegenhaltungen :
EP-A- 0 257 444
DE-A- 1 262 976
US-A- 2 995 278

(56) Entgegenhaltungen :
WPIL, File Supplier, AN 88-010735, Derwent Publications Ltd., (London, GB) & JP-A-62273912 )Kao Corp.) 28.11.87
WPIL, File Supplier, AN 90-026698, Derwent Publications Ltd., (London, GB) & JP-A-1305023 (Toyo Aerosol Kogyo) 8.12.89

(73) Patentinhaber : Wella Aktiengesellschaft
Berliner Allee 65
W-6100 Darmstadt (DE)

(72) Erfinder : LANG, Günther
Auf der Roten Erde 10 B
W-6107 Reinheim 5 (DE)
Erfinder : HERFURT, Ulrich
Smetana Weg 1
W-6500 Mainz 31 (DE)
Erfinder : PETRI, Harald
Gronaustrasse 3
W-6200 Wiesbaden (DE)

## Beschreibung

Gegenstand der Erfindung ist ein mit komprimierten Gasen oder mit mechanisch betriebenen Sprühvorrichtungen versprühbares, von verflüssigten Treibmitteln freies Lösungsmittelgemisch. Das Lösungsmittelgemisch besteht aus einem einphasigen Gemisch von Alkohol, Wasser und bestimmten Alkanen. Gegenstand der Erfindung sind zudem kosmetische Mittel, die dieses Lösungsmittelgemisch enthalten.

Die für sprühbare kosmetische Zubereitungen gebräuchlicherweise eingesetzten Lösungsmittel sind niedere Alkohole, insbesondere Ethylalkohol und Isopropylalkohol. Diese Alkohole zeichnen sich durch ein gutes Lösevermögen für die meisten in kosmetischen Mitteln verwendeten Wirksubstanzen, gute Mischbarkeit mit anderen Lösungsmittel sowie durch eine geringe Toxizität und einen angenehmen Geruch aus.

Allerdings unterliegen die genannten Alkohole, insbesondere Ethylalkohol, in vielen Ländern erheblicher Besteuerung, was zu hohen Kosten bei Produkten mit hohem Alkoholanteil führt. Zudem haben reinen Alkohol als Lösungsmittel enthaltende kosmetische Mittel sehr kurze Trocknungszeiten. Diese kurzen Trocknungszeiten können sich insbesondere bei Haarstyling-Produkten, bei denen die Frisurenformung nach dem Aufsprühen des Mittels erfolgt, nachteilig auswirken.

Zur Herabsetzung der Alkoholmenge wird in Lösungsmitteln für kosmetische Zubereitungen häufig Wasser mitverwendet. Der Einsatz von mehr als 5 Gewichtsprozent Wasser führt jedoch im allgemeinen durch die geringe Flüchtigkeit dieses Lösungsmittels zu relativ langen Trocknungszeiten und einer Verschlechterung des Sprühbildes.

Werden anstelle des Wassers leichter flüchtige Lösungsmittel, wie zum Beispiel geruchsarme niedere Alkane, als Teilersatz für die Alkohole verwendet, ergeben sich, insbesondere bei zur Haar- und Frisurenfestigung dienenden Mitteln, ebenfalls Nachteile. Infolge zu schneller Verdunstung wird das erwünschte Verlaufen des Produktes auf dem Haar beeinträchtigt. Daraus ergeben sich geringere Festigungseffekte und eine ungleichmäßige Produktverteilung.

Alkane, insbesondere Propan, Butan und Isopentan, sind bisher als Treibmittel für kosmetische Mittel, die ein Alkohol-Wasser-Gemisch als Lösungsmittel enthalten, bekannt. So sind aus der DE-OS 36 27 970 Aerosol-Haarspray-Zubereitungen bekannt, die Propan, Butan oder Isopentan als Treibmittel und Alkohol-Wasser-Gemische als Lösungsmittel enthalten können.

Aus ökologischen Gründen ist die Verwendung sprühbarer kosmetischer Mittel, welche frei von verflüssigten Treibmitteln sind, den noch weit verbreiteten, verflüssigte rase als Treibmittel enthaltenden Aerosol-Präparaten jedoch vorzuziehen.

Es bestand daher die Aufgabe, ein einphasiges, durch einen reduzierten Alkoholgehalt kostengünstiges Lösungsmittel für kosmetische Mittel, welche mit komprimierten Gasen oder mit mechanisch betriebenen Sprühvorrichtungen versprühbar und frei von verflüssigten Treibmitteln sind, bereitzustellen, durch das die aus dem Stand der Technik bekannten Nachteile üblicher Lösungsmittel hinsichtlich Sprühbild und Verdunstungsverhalten vermieden werden.

Es wurde nun gefunden, daß ein einphasiges Lösungsmittelgemisch für kosmetische Mittel, welches mit komprimierten Gasen oder mit mechanisch betriebenen Sprühvorrichtungen versprühbar und frei von verflüssigten Treibmitteln ist, auf der Basis von

(A) 50 bis 94,5 Gewichtsprozent eines $C_2$-bis $C_4$-Alkohols oder Gemischen dieser Alkohole,
dadurch gekennzeichnet, daß es
(B) 5 bis 30 Gewichtsprozent Wasser
und
(C) 0,5 bis 45 Gewichtsprozent mindestens eines niederen Alkans, ausgewählt aus n-Pentan, n-Hexan und Isohexan

enthält, wobei das Verhältnis der Gewichtsanteile der komponente (B) zu den Gewichtsanteilen der Komponente (C) 0,1:1 bis 10:1 beträgt, die gestellte Aufgabe in hervorragender Weise löst.

Die das erfindungsgemäße Lösungsmittelgemisch enthaltenden kosmetischen Mittel zeigen auch bei einem Wassergehalt von über 10 Gewichtsprozent keine Verschlechterung des Sprühbildes. Durch die erreichte Herabsetzung der Produktviskosität gegenüber vergleichbaren Ethanol-Wasser-Gemischen wird zudem eine deutlich verbesserte Versprühbarkeit des das erfindungsgemäße Lösungsmittelgemisch enthaltenden kosmetischen Mittels mit mechanischen Sprühsystemen erreicht.

Das erfindungsgemäße Lösungsmittelgemisch zeigt annähernd die vorteilhaften Gebrauchseigenschaften reiner Alkohole, ist jedoch kostengünstiger als diese und hat zudem den Vorteil einer verbesserten Produktstabilität, da durch den relativ hohen Dampfdruck der verwendeten Alkane Sauerstoff aus dem Sprühbehälter verdrängt wird.

Ein weiterer Vorteil der das erfindugsgemäße Lösungsmittelgemisch enthaltenden kosmetischen Mittel ist, daß sie frei von verflüssigten Treibmitteln wie Halogenkohlenwasserstoffen oder als Treibmittel geeigneten Alkanen, wie zum Beispiel Propan, Butan, Isobutan, Isopentan, 2,2-Dimethylbutan oder Gemische derselben, vorliegen. Die nachteiligen Eigenschaften der mit verflüssigten Treibmitteln betriebenen Sprays, wie zum Beispiel mögliche Umweltschäden (Abbau der Ozonschicht), werden vermieden.

Unter mechanischen Sprühvorrichtungen sind solche Vorrichtungen zu verstehen, welche das Versprühen einer Flüssigkeit ohne Verwendung eines verflüssigten Treibmittels ermöglichen. Als geeignete mechanische Sprühvorrichtung kann beispielsweise eine Sprühpumpe oder ein mit einem Sprühventil versehener elastischer Behälter, in den das das vorstehend beschriebene Lösungsmittel enthaltende kosmetische Mittel unter Druck abgefüllt wird, wobei sich der elastische Behälter ausdehnt und aus dem sich das Mittel infolge der Kontraktion des elastischen Behälters bei öffnen des Sprühventils kontinuierlich entnehmen läßt, verwendet werden.

Unter komprimierten Gasen sind Gase zu verstehen, die im Lösungsmittelgemisch unlöslich oder wenig löslich sind und sich bei den üblichen geringen Behälterdrücken von 1 bis 5 bar nicht verflüssigen lassen, wie zum Beispiel Stickstoff, Kohlendioxid, Distickstoffoxid oder Preßluft. Zum Versprühen des erfindungsgemäßen Lösungsmittelgemischs oder der das Lösungsgemisch enthaltenen kosmetischen Mittel ist die Verwendung mechanischer Sprühvorrichtungen bevorzugt.

Als niedere Alkane werden n-Pentan, n-Hexan und Isohexan, jeweils allein oder im Gemisch, in dem erfindungsgemäßen Lösungsmittelgemisch eingesetzt. Die niedrigen Alkane sollen in dem Lösungsmittelgemisch vorzugsweise in einer Menge von 3 bis 20 Gewichtsprozent enthalten sein.

Als $C_2$- bis $C_4$-Alkohole werden vorzugsweise Ethylalkohol, n-Propylalkohol und Isopropylalkohol, allein oder im Gemisch, verwendet, wobei Ethylalkohol bevorzugt ist. Die $C_2$- bis $C_4$-Alkohole oder deren Gemische sind in dem erfindungsgemäßen Lösungsmittelgemisch in einer Menge von 50 bis 94,5 Gewichtsprozent, vorzugsweise 60 bis 80 Gewichtsprozent enthalten.

Das erfindungsgemäße, einphasige Lösungsmittelgemisch kann zusätzlich physiologisch verträgliche $C_3$- bis $C_6$-Ketone, wie zum Beispiel Aceton, $C_3$- bis $C_8$-Glykolether, wie zum Beispiel Propylenglykolmonomethylether, oder deren Gemische in einer Menge von 1 bis 30 Gewichtsprozent enthalten.

In der bevorzugten Auführungsform soll das erfindungsgemäße einphasige Lösungmittelgemisch 10 bis 25 Gewichtsprozent der Komponente (B), des Wassers, besonders bevorzugt 15 bis 25 Gewichtsprozent und die Komponente (C) in eienr Menge von 3 bis 20 Gewichtsprozent, jeweils bezogen auf die Gesamtmenge des Lösungsmittelgemisches, enthalten. Das Verhaltnis der Gewichtsanteile der Komponente (B) zu den Gewichtsanteilen der Komponente (C) soll in der bevorzugten Ausführungsform des erfindungsgemäßen Lösungsmittelgemischs 0,5:1 bis 10:1, besonders bevorzugt 0,5:1 bis 5:1 betragen.

Das erfindungsgemäße sprühbare Lösungsmittelgemisch kann in kosmetischen Mitteln eingesetzt werden, die zur Behandlung der menschlichen Haut oder zur Behandlung der Haare verwendet werden. Gegenstand der vorliegenden Anmeldung sind daher kosmetische Mittel zur Behandlung der Haut oder der Haare mit einem Gehalt an dem erfindungsgemäßen Lösungsmittelgemisch. Das Lösungsmittelgemisch soll in den kosmetischen Mitteln in einer Menge von 50 bis 99 Gewichtsprozent, vorzugsweise jedoch in einer Menge von 70 bis 99 Gewichtsprozent, enthalten sein.

Das erfindungsgemäße Lösungsmittelgemisch kann in kosmetischen Mitteln zur Aufbringung auf die menschliche Haut, zum Beispiel für deodorierende Mittel auf der Basis bakterizider Wirkstoffe und/oder zur Pflege mittels rückfettenden Substanzen oder als Insekten-Repellent, enthalten sein.

Als Bestandteil von auf die Haare aufzubringenden kosmetischen Mitteln, wie zum Beispiel Mitteln zur Frisurenfestigung mittels polymerer Substanzen und/oder zur Glanzgebung und Haarpflege mittels öliger, spreitender Substanzen, ist das erfindungsgemäße Lösungsmittelgemisch in hervorragender Weise geeignet.

Das erfindungsgemäße Lösungsmittelgemisch kann insbesondere für Haarfestigungspräparate, die mittels mechanischer Sprühpumpen oder mittels mit komprimierten Gasen betriebener Sprühvorrichtungen auf das frisierte Haar aufgebracht werden, eingesetzt werden.

Ein weiterer Vorteil des erfindungsgemäßen Lösungsmittelgemisches liegt in der Verkürzung der Gesamttrocknungszeit des das erfindungsgemäße Lösungsmittelgemisch enthaltenden, versprühten Haarfestigungspräparates. Zudem resultiert aus der durch die Verwendung des erfindungsgemäßen Lösungsmittelgemisches erreichten Herabsetzung der Produktviskosität der zur Haarfestigung dienenden Polymerlösungen eine deutlich verbesserte Verspühbarkeit dieser Haarfestigungspräparate.

Selbstverständlich kann das erfindungsgemäße Mittel für Haar- und Hautbehandlungsmittel übliche kosmetische Zusatzstoffe enthalten, sofern sie in den erfindungsgemäßen Lösungsmittelgemisch löslich sind.

Als geeignete kosmetische Zusatzstoffe in diesen Mitteln können beispielsweise anionische, kationische, amphotere oder nicht ionische Netzmittel und Emulgatoren, wie zum Beispiel $C_{12}$- bis $C_{18}$-Alkylethersulfat, Alkyltrimethylammoniumsalze, Alkylpyridiniumsalze, Carboxyderivate von Imidazol, N-Alkylsulfobetain oder Polyglycerylether von gesättigten oder ungesättigten Fettalkoholen und Alkylphenolen in einer Menge von etwa 0,01 bis 3 Gewichtsprozent, ferner Konservierungsstoffe, wie zum Beispiel Salicylsäure oder Mandelsäure in einer Menge von 0,01 bis 0,7 Gewichtsprozent, sowie Antischuppenwirkstoffe, wie zum Beispiel Zink-Pyridinthion, kosmetische Farbstoffe, wie zum Beispiel Fluorescein-Natriumsalz, weiterhin haar-und hautpflegende Wirkstoffe, wie zum Beispiel Fettsäurester, Fettalkohole, Fettsäureglyceride, Chitosanderivate, Lanolinderivate oder Pantothensäure in einer Menge von etwa 0,01 bis 3 Gewichtsprozent, sowie ferner Komplexbildner, Schaumstabilisatoren, Puffersubstanzen, Lichtschutzmittel oder Parfümöle in einer Menge von etwa 0,01 - 0,8 Gewichtsprozent enthalten sein.

Die nachfolgenden Beispiele sollen den Gegenstand der Erfindung näher erläutern.

**Beispiele**

Beispiel 1:     Non-Aerosol Haarspray

| | |
|---|---|
| 5,0 g | Octylacrylamid/Acrylsäure-Polymer neutralisiert mit Triisopropanolamin |
| 69,6 g | Ethylalkohol |
| 10,0 g | Wasser |
| 15,0 g | n-Pentan |
| 0,4 g | Parfümkomposition |
| 100,0 g | |

Beispiel 2:     Non-Aerosol-Haarspray

| | |
|---|---|
| 7,0 g | Polyvinylpyrrolidon/Vinylacetat-Copolymer |
| 67,6 g | Isopropylalkohol |
| 15,0 g | Wasser |
| 10,0 g | n-Hexan |
| 0,1 g | Phenylmethylpolysiloxan |
| 0,3 g | Parfümkomposition |
| 100,0 g | |

Beispiel 3:     Non-Aerosol-Körperdeodorant

| | |
|---|---|
| 68,5 g | Ethylalkohol |
| 25,0 g | Wasser |
| 5,0 g | Hexan-Isomerengemisch |
| 0,5 g | Emulgator |
| 1,0 g | Parfümkomposition |
| 100,0 g | |

4

**Vergleichsversuche mit Haarfestigungspräparaten**

1. Einsetzen der Klebephase und Gesamttrocknungszeit

Die verglichenen Haarfestigungspräparate enthielten jeweils 10 Gewichtsprozent Polyvinylpyrrolidon/Vinylacetat als Festigungssubstanz und wurden mittels einer mechanischen Sprühpumpe mit Vordruck-Aufbau und einer Ausbringmenge von ca. 0,14 ml pro Hub auf frisiertes Haar aufgebracht. Im Halbseitenversuch mit jeweils 10 Pumphüben pro Seite, bei einem Sprühabstand von 25 cm, wurden folgende Verdunstungs-, beziehungsweise Trocknungs-Verläufe gefunden. In der Tabelle sind Mittelwerte aus jeweils vier Untersuchungen wiedergegeben.

```
------------------------------------------------------

                                 Einsetzen    Gesamttrock-

                                 der Klebe-   nungszeit


                                 phase nach
------------------------------------------------------
Präparat A mit

mit ausschließlich

Ethylalkohol als                              1 min 30 s

Lösungsmittel         12-20 s                 bis 2 min 30 s


Präparat B

mit Lösungsmittelge-

misch Ethylalkohol/                           2 min bis

Wasser 90/10          20-38 s                 3 min 40 s


Präparat C

mit Lösungsmittelge-

misch Ethylalkohol/                           2 min bis

Wasser/n-Pentan 80/10/10  10-20 s            2 min 40 s
```

Das das erfindungsgemäße Lösungsmittelgemisch enthaltende Präparat C zeigt gegenüber Präparat B eine deutlich früher einsetzende Klebephase und eine verkürzte Gesamttrocknungszeit.

2. Kinematische Viskosität

Bei Messungen der kinematischen Viskosität verschiedener Haarfestigungspräparate, die jeweils 10 Gewichtsprozent Polyvinylpyrrolidon/Vinylacetat als Festigungssubstanz enthielten, wurden die folgenden Werte als Mittelwert aus fünf Bestimmungen erhalten.

|  | kinematische Viskosität $(mm^2/s)$ |
|---|---|
| Präparat A1 mit Lösungsmittelgemisch Ethyl-alkohol/Wasser 90/10 | 10,8 |
| Präparat A2 mit Lösungsmittelgemisch Ethyl-alkohol/Wasser 80/20 | 13,8 |
| Präparat B mit Lösungsmittelgemisch Ethyl-alkohol/Wasser/n-Pentan 80/10/10 | 9,4 |

Die Messungen wurden mit einem Ubbelohde-Viskosimeter Typ 501 103 (Kapillare 1c, innerer Kappilardurchmesser: 0,84 mm) bei 20 Grad Celsius durchgeführt.

Wie sich aus der Tabelle ersehen läßt, ist die Viskosität des das erfindungsgemäße Lösungsmittelgemisch enthaltenden Präparates B im Vergleich zu den die entsprechenden Alkohol-Wasser Gemische enthaltenden Präparaten A1 und A2 deutlich kleiner, was die verbesserte Versprühbarkeit der erfindungsgemäßen Mittel bewirkt.

Die Prozentangaben sollen, sofern nicht anders angegeben, Gewichtsprozent darstellen.

**Patentansprüche**

1. Einphasiges Lösungsmittelgemisch für kosmetische Mittel, welches mit komprimierten Gasen oder mit mechanisch betriebenen Sprühvorrichtungen versprühbar und frei von verflüssigten Treibmitteln ist, auf der Basis von

   (A) 50 bis 94,5 Gewichtsprozent eines $C_2$-bis $C_4$-Alkohols oder Gemischen dieser Alkohole, dadurch gekennzeichnet, daß es
   (B) 5 bis 30 Gewichtsprozent Wasser
   und
   (C) 0,5 bis 45 Gewichtsprozent mindestens eines niederen Alkans, ausgewählt aus n-Pentan, n-Hexan und Isohexan
   enthält, wobei das Verhältnis der Gewichtsanteile der Komponente (B) zu den Gewichtsanteilen der Komponente (C) 0,1:1 bis 10:1 beträgt.

2. Lösungsmittelgemisch nach Anspruch 1, dadurch gekennzeichnet, daß die Komponente (B) in einer Menge von 10 bis 25 Gewichtsprozent und die Komponente (C) in einer Menge von 3 bis 20 Gewichtsprozent enthalten ist.

3. Lösungsmittelgemisch nach Anspruch 1, dadurch gekennzeichnet, daß die Komponente (B) in einer Menge von 15 bis 25 Gewichtsprozent enthalten ist.

4. Lösungsmittelgemisch nach einem der Ansprüche 1 bis 3, dadurch gekennzeichnet, daß das Verhältnis der Gewichtsanteile der Komponente (B) zu den Gewichtsanteilen der Komponente (C) 0,5:1 bis 10:1 beträgt.

**5.** Lösungsmittelgemisch nach einem der Ansprüche 1 bis 3 dadurch gekennzeichnet, daß das Verhältnis der Gewichtsanteile der Komponente (B) zu den Gewichtsanteilen der Komponente (C) 0,5:1 bis 5:1 beträgt.

**6.** Lösungsmittelgemisch nach einem der Ansprüche 1 bis 5, dadurch gekennzeichnet, daß es 60 bis 80 Gewichtsprozent $C_2$- bis $C_4$-Alkohole oder deren Gemisch enthält.

**7.** Lösungsmittelgemisch nach einem der Ansprüche 1 bis 6, dadurch gekennzeichnet, daß der $C_2$-Alkohol Ethylalkohol ist.

**8.** Lösungsmittelgemisch nach einem der Ansprüche 1 bis 7, dadurch gekennzeichnet, daß es $C_3$- bis $C_6$-Ketone oder $C_3$- bis $C_8$-Glykolether oder deren Gemisch enthält.

**9.** Kosmetisches Mittel zur Behandlung der Haut oder der Haare, dadurch gekennzeichnet, daß es 50 bis 99 Gewichtsprozent eines Lösungsmittelgemisches nach einem der Anprüche 1 bis 8 enthält.

**10.** Mittel nach Anspruch 9, dadurch gekennzeichnet, daß es das Lösungsmittelgemisch in einer Menge von 70 bis 99 Gewichtsprozent enthält.

**11.** Mittel nach Anspruch 9, dadurch gekennzeichnet daß es ein deodorierendes Mittel ist.

**12.** Mittel nach Anspruch 9, dadurch gekennzeichnet, daß es ein Insekten-Repellent ist.

**13.** Mittel nach Anspruch 9, dadurch gekennzeichnet, daß es ein Mittel zur Frisurenfestigung ist.

## Claims

**1.** Single-phase solvent mixture for cosmetic compositions which can be sprayed by means of compressed gases or mechanically driven spray devices and is free from liquified propellants, based on
(A) 50 to 94.5 weight % of a $C_2$ to $C_4$ alcohol or mixtures of these alcohols,
characterised in that it contains
(B) 5 to 30 weight % of water and
(C) 0.5 to 45 weight % of at least one lower alkane selected from n-pentane, n-hexane and isohexane,
the ratio of the percentage by weight of component (B) relative to the percentage by weight of component (C) being 0.1:1 to 10:1.

**2.** Solvent mixture according to Claim 1, characterised in that component (B) is present in an amount from 10 to 25 weight % and component (C) is present in an amount from 3 to 20 weight %.

**3.** Solvent mixture according to Claim 1, characterised in that component (B) is present in an amount from 15 to 25 weight %.

**4.** Solvent mixture according to any one of Claims 1 to 3, characterised in that the ratio of the percentages by weight of component (B) relative to the percentages by weight of component (C) is 0.5:1 to 10:1.

**5.** Solvent mixture according to any one of Claims 1 to 3, characterised in that the ratio of the percentages by weight of component (B) relative to the percentages by weight of component (C) is 0.5:1 to 5:1.

**6.** Solvent mixture according to any one of Claims 1 to 5, characterised in that it contains 60 to 80 weight % of $C_2$ to $C_4$ alcohols or a mixture thereof.

**7.** Solvent mixture according to any one of Claims 1 to 6, characterised in that the $C_2$ alcohol is ethyl alcohol.

**8.** Solvent mixture according to any one of Claims 1 to 7, characterised in that it contains $C_3$ to $C_6$ ketones or $C_3$ to $C_8$ glycolether or a mixture thereof.

**9.** Cosmetic composition for treating the skin or hair, characterised in that it contains 50 to 99 weight % of a solvent mixture according to any one of Claims 1 to 8.

10. Composition according to Claim 9, characterised in that it contains the solvent mixture in an amount of between 70 and 99 weight %.

11. Composition according to Claim 9, characterised in that it is a deodorising composition.

12. Composition according to Claim 9, characterised in that it is an insect repellent.

13. Composition according to Claim 9, characterised in that it is a composition for setting hair styles.

## Revendications

1. Mélange de diluant monophasique pour produit cosmétique, pulvérisable à l'aide de gaz comprimés ou à l'aide de dispositifs de pulvérisation mécaniques et exempt de produits propulseur liquéfié, à base de :
(a) 50 à 94,5 pourcent en poids d'un alcool en $C_2$ à $C_4$ ou d'un mélange de ces alcools,
caractérisé en ce qu'il contient
(b) de 5 à 30 pourcent en poids d'eau
et
(c) de 0,5 à 45 pourcent en poids d'au moins un alcane inférieur, sélectionné parmi le n-pentane, le n-hexane et l'isohexane,
le rapport entre les proportions en poids du composant (B) et les proportions en poids du composant (C) étant de 0,1 : 1 à 10 : 1.

2. Mélange de diluant selon la revendication 1, caractérisé en ce que le composant (B) est contenu en une quantité de 10 à 25 pourcent en poids et le composant (C) en une quantité de 3 à 20 pourcent en poids.

3. Mélange de diluant selon la revendication 1, caractérisé en ce que le composant (B) est contenu en une quantité de 15 à 25 pourcent en poids.

4. Mélange de diluant selon l'une des revendications 1 à 3, caractérisé en ce que le rapport entre les proportions en poids du composant (B) et les proportions en poids du composant (C) étant de 0,5 : 1 à 10 : 1.

5. Mélange de diluant selon l'une des revendications 1 à 3, caractérisé en ce que le rapport entre les proportions en poids du composant (B) et les proportions en poids du composant (C) étant de 0,5 : 1 à 5 : 1.

6. Mélange de diluant selon l'une des revendications 1 à 5, caractérisé en ce qu'il contient de 60 à 80 pourcent en poids d'alcools en $C_2$ à $C_4$ ou de leur mélange.

7. Mélange de diluant selon l'une des revendications 1 à 6, caractérisé en ce que l'alcool en $C_2$ est l'alcool éthylique.

8. Mélange de diluant selon l'une des revendications 1 à 7, caractérisé en ce qu'il contient une cétone en $C_3$ à $C_6$ ou un glycoléther en $C_3$ à $C_8$ ou leur mélange.

9. Produit cosmétique, pour le traitement de la peau ou des cheveux, caractérisé en ce qu'il contient de 50 à 99 pourcent en poids d'un mélange de diluant selon l'une des revendications 1 à 8.

10. Produit selon la revendication 9, caractérisé en ce qu'il content le mélange de diluant en une quantité de 70 à 99 pourcent en poids.

11. Produit selon la revendication 9, caractérisé en ce qu'il est un produit déodorant.

12. Produit selon la revendication 9, caractérisé en ce qu'il est un produit répulsif des insectes.

13. Produit selon la revendication 9, caractérisé en ce qu'il est un produit de fixation de la coiffure.